# EUROPEAN PATENT APPLICATION

(11) **EP 3 590 443 A1**
(43) Date of publication of application: **08.01.2020**
(21) Application number: 19184099.0
(22) Date of filing: 03.07.2019
(51) Int. Cl.: A61B 17/12

(54) **INDWELLING DEVICE**

(30) Priority: 06.07.2018 JP 2018129512
(71) Applicant: Asahi Intecc Co., Ltd., Seto-shi, Aichi 489-0071 (JP)
(72) Inventor: KIKUCHI, Daisuke, Meguro-ku, Tokyo 152-0021 (JP); NAMIMA, Satoshi, Seto-shi, Aichi 489-0071 (JP); TSUKAMOTO, Toshihiko, Seto-shi, Aichi 489-0071 (JP)
(74) Representative: Winter, Brandl, Fürniss, Hübner, Röss, Kaiser, Polte - Partnerschaft mbB

(57) **Abstract**

[Problem to be Solved] To provide an indwelling device capable of aspirating a lesion easily into the indwelling device, and preventing leakage of the lesion to the outside and necrotizing the lesion reliably after hold of the lesion in the indwelling device, and to provide an indwelling device capable of preventing leakage of a lesion to the outside and holding the lesion reliably even if the lesion is relatively large in volume.

[Solution to Problem] An indwelling device 1 is configured with a hollow tubular body, and includes a device body 5 and a device tip portion 3 connected to the tip of the device body 5 and made of an elastic member.

## Description

### Technical Field

The present invention relates to an indwelling device for aspirating a lesion at a body tissue of a patient such as a vessel, an intestinal wall or a stomach wall, and then compressing and removing the aspirated lesion.

### Background Art

According to a conventional method of ligation of a wound on an affected part removed by an endoscopic operation, a medical grasping tool housed in a tube such as a sheath is transferred to the vicinity of the wound of a patient by remote control from a hand side of a tube. The transferred medical grasping tool is operated to ligate the wound of the patient. Then, the medical grasping tool is placed in the body of the patient.

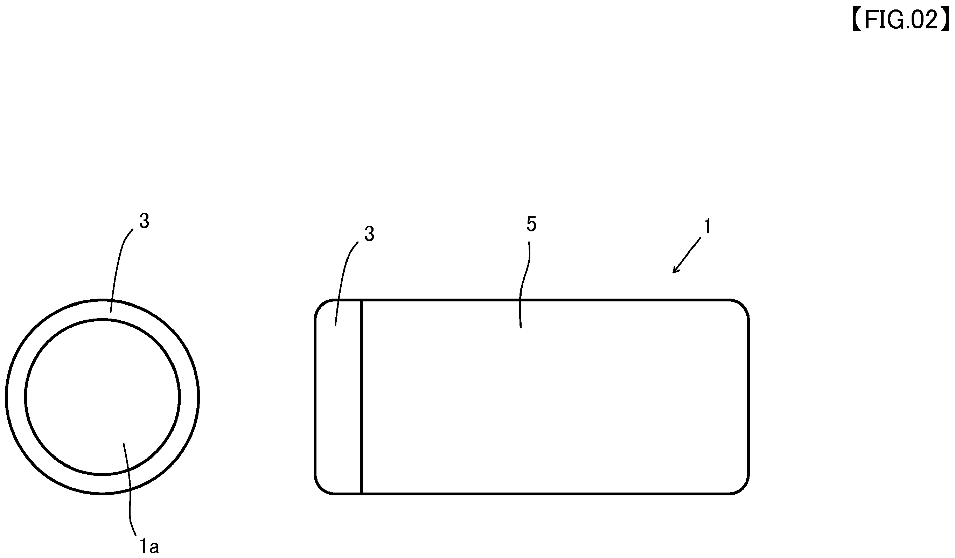

For example, patent literature 1 describes a medical device for ligation of a wound on an affected part removed by an endoscopic operation. This medical device includes an external tube 10, an internal tube 20 inserted into the interior of the external tube, an operation wire 30 inserted into the interior of the internal tube 20, an operation ring 31 provided at the base end of the operation wire 30 and projecting from the base ends of the external tube 10 and the internal tube 20, and a medical grasping tool 40 inserted and fitted in the tip of the internal tube 20 and connected to the tip of the operation wire 30 so as to be removable from the tip of the operation wire 30 (see Fig. 2, for example).

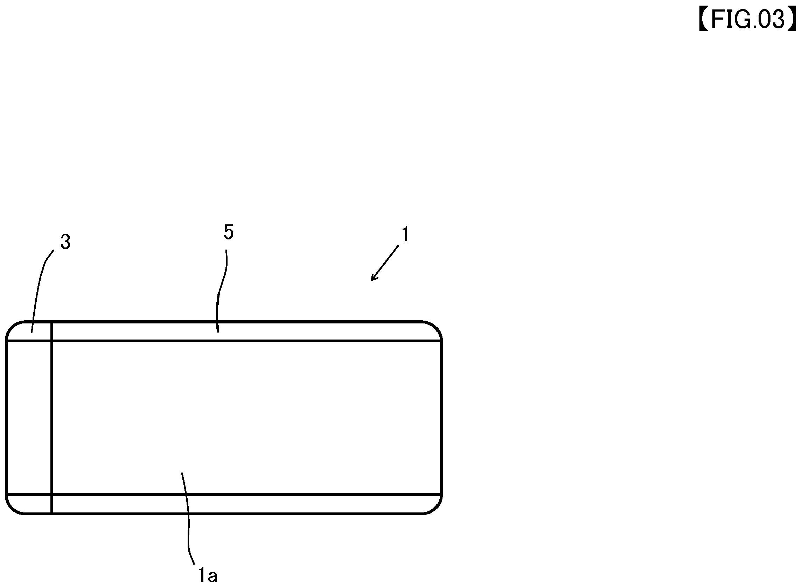

In this medical device, the external tube 10 is operated to transfer the medical grasping tool 40 to the vicinity of a wound of a patient. The transferred medical grasping tool 40 is brought into abutting contact with the wound of the patient by operating the internal tube 20, the operation wire 30, and the operation ring 31 to achieve ligation of the wound of the patient (see Figs. 3 to 8, for example).

### Citation list

### Patent Literature

Patent Literature 1: Japanese Patent Application Publication No. 2013-244054

### Summary of Invention

### Technical Problem

For digestive disorders such as disorders of colon diverticula, gastric varices or esophageal varices, for example, development of a medical device appropriate for such disorders has been expected.

While use of a medical grasping tool such as that described in patent literature 1 has conventionally been attempted, such treatment has failed to efficiently cure colon diverticula jetting out externally from intestinal tracts. Such treatment has also caused difficultly in placing the medical grasping tool in vessels successively for curing gastric varices or esophageal varices.

Even after the medical device is placed in a body, there arises necessity to hold a lesion reliably in the medical device and to eventually necrotize a lesion reliably and discharge the necrotized lesion and the medical device together to the outside of the body of a patient safely.

This present invention has been made in response to the foregoing problems of the conventional technique, and is intended to provide an indwelling device capable of aspirating a lesion easily into the indwelling device, and preventing leakage of the lesion to the outside and necrotizing the lesion reliably after hold of the lesion in the indwelling device. The present invention is also intended to provide an indwelling device capable of preventing leakage of a lesion to the outside and holding the lesion reliably even if the lesion is relatively large in volume.

### Solution to Problem

It is characterized in that, to solve the foregoing problems, an indwelling device according to a first aspect of the present invention is configured with a hollow tubular body. The hollow tubular body has an end portion made of an elastic member.

A second aspect of the present invention is characterized in that, in the indwelling device according to the first aspect, the hollow tubular body includes a diameter-increased portion provided closer to a different end than the end portion and having an inner diameter larger than the inner diameter of the end portion.

A third aspect of the present invention is characterized in that, in the indwelling device according to the second aspect, the hollow tubular body includes a diameter-reduced portion provided closer to the different end than the diameter-increased portion and having an inner diameter smaller than the inner diameter of the diameter-increased portion.

A fourth aspect of the present invention is characterized in that, in the indwelling device according to the second or third aspect, the hollow tubular body has an outer shape formed into a streamlined shape as a whole.

A fifth aspect of the present invention is characterized in that, in the indwelling device according to any one of the second to fourth aspects, the diameter-increased portion includes a braid and a resin portion covering the braid.

A sixth aspect of the present invention is characterized in that, in the indwelling device according to the fifth aspect, the braid forming the diameter-increased portion is at least partially made of a radiopaque material.

A seventh aspect of the present invention is characterized in that, in the indwelling device according to any one of the second to sixth aspects, the diameter-increased portion is reducible in diameter in a housing in which the indwelling device is accommodated and increases in diameter automatically when the indwelling device is separated from the housing.

An eighth aspect of the present invention is characterized in that, in the indwelling device according to any one of the first to seventh aspects, the indwelling device comprises a check valve provided at the end portion of the hollow tubular body.

A ninth aspect of the present invention is characterized in that, in the indwelling device according to any one of the first to eighth aspects, the indwelling device comprises a check valve provided at an end portion on the opposite side of the end portion of the hollow tubular body.

A tenth aspect of the present invention is characterized in that, in the indwelling device according to any one of the first to ninth aspects, the elastic member is polyurethane elastomer.

### Advantageous Effects of Invention

The indwelling device according to the first aspect of the present invention is configured with the hollow tubular body and the hollow tubular body has an end portion made of an elastic member. Thus, after the end portion of the indwelling device is brought into abutting contact with a lesion and the lesion is aspirated from the different end of the indwelling device into the indwelling device, the indwelling device can reliably be placed in a body using the elastic force of the elastic member of the end portion and the lesion can reliably be necrotized using the elastic force of the elastic member of the end portion.

According to the second aspect of the present invention, in the indwelling device according to the first aspect, the hollow tubular body includes the diameter-increased portion provided closer to the different end than the end portion and having an inner diameter larger than the inner diameter of the end portion. Thus, even in the case of a lesion having a large volume, the indwelling device can be placed in a body reliably using the elastic force of the elastic member and the lesion can be necrotized reliably using the elastic force of the elastic member of the end portion.

According to the third aspect of the present invention, in the indwelling device according to the second aspect, the hollow tubular body includes the diameter-reduced portion provided closer to the different end than the diameter-increased portion and having an inner diameter smaller than the inner diameter of the diameter-increased portion. In addition to achieving the effect of the indwelling device according to the second aspect, this achieves the effect of holding a lesion reliably inside the indwelling device.

According to the fourth aspect of the present invention, in the indwelling device according to the second or third aspect, the hollow tubular body has an outer shape formed into a streamlined shape as a whole. In addition to achieving the effect of the indwelling device according to the second or third aspect, this achieves the effect of inserting the indwelling device easily into a catheter, for example, for guiding the indwelling device into a body.

According to the fifth aspect of the present invention, in the indwelling device according to any one of the second to fourth aspects, the diameter-increased portion includes the braid and the resin portion covering the braid. In addition to achieving the effect of the indwelling device according to any one of the second to fourth aspects, this achieves the effect of preventing break of a lesion even if the lesion is aspirated into the diameter-increased portion in a compressed state and holding the lesion reliably inside the indwelling device.

According to the sixth aspect of the present invention, in the indwelling device according to the fifth aspect, the braid forming the diameter-increased portion is at least partially made of a radiopaque material. In addition to achieving the effect of the indwelling device according to the fifth aspect, this achieves the effect of visually recognizing the position of the indwelling device reliably by radioscopy.

According to the seventh aspect of the present invention, in the indwelling device according to any one of the second to sixth aspects, the diameter-increased portion is reducible in diameter in the housing in which the indwelling device is accommodated and increases in diameter automatically when the indwelling device is separated from the housing. In addition to achieving the effect of the indwelling device according to any one of the second to sixth aspects, this further prevents leakage of a lesion to the outside of the indwelling device and allows the lesion to be held reliably inside the indwelling device.

According to the eighth aspect of the present invention, in the indwelling device according to any one of the first to seventh aspects, the indwelling device comprises the check valve provided at the end portion of the hollow tubular body. In addition to achieving the effect of the indwelling device according to any one of the first to seventh aspects, this further prevents leakage of a lesion to the outside of the indwelling device and allows the lesion to be held reliably inside the indwelling device.

According to the ninth aspect of the present invention, in the indwelling device according to any one of the first to eighth aspects, the indwelling device comprises the check valve provided at an end portion on the opposite side of the end portion of the hollow tubular body. In addition to achieving the effect of the indwelling device according to any one of the first to eighth aspects, this further prevents leakage of a lesion to the outside of the indwelling device and allows the lesion to be held reliably inside the indwelling device.

According to the tenth aspect of the present invention, in the indwelling device according to any one of the first to ninth aspects, the elastic member is polyurethane elastomer. In addition to achieving the effect of the indwelling device according to any one of the first to ninth aspects, this achieves the effect of bringing the indwelling device into abutting contact with a lesion without damaging the lesion.

### Brief Description of Drawings

Fig. 1 shows an overall schematic view of a medical instrument for placement of an indwelling device of the present invention in a body;
Fig. 2 shows a front view and a side view of an indwelling device of a first embodiment;
Fig. 3 shows a side sectional view of the indwelling device of the first embodiment;
Fig. 4 shows an enlarged view of a section at an area A in Fig. 1 and shows a view at a first stage for explaining a step of placing the indwelling device of the first embodiment in the body of a patient;
Fig. 5 shows an enlarged view of the section at the area A in Fig. 1 and shows a view at a second stage for explaining a step of placing the indwelling device of the first embodiment in the body of the patient;
Fig. 6 shows an enlarged view of the section at the area A in Fig. 1 and shows a view at a third stage for explaining a step of placing the indwelling device of the first embodiment in the body of the patient;
Fig. 7 shows an enlarged view of the section at the area A in Fig. 1 and shows a view at a fourth stage for explaining a step of placing the indwelling device of the first embodiment in the body of the patient;
Fig. 8 shows an enlarged view of the section at the area A in Fig. 1 and shows a view at a final stage for explaining a step of placing the indwelling device of the first embodiment in the body of the patient;
Fig. 9 shows a side sectional view of an indwelling device of a second embodiment;
Fig. 10 shows a front view and a side view of an indwelling device of a third embodiment;
Fig. 11 shows a side sectional view of the indwelling device of the third embodiment;
Fig. 12 shows a side sectional view of an indwelling device of a fourth embodiment;
Fig. 13 shows a front view and a side view of an indwelling device of a fifth embodiment;
Fig. 14 shows a side sectional view of the indwelling device of the fifth embodiment;
Fig. 15 shows a front view and a side view of an indwelling device of a sixth embodiment;
Fig. 16 shows a side sectional view of the indwelling device of the sixth embodiment;
Fig. 17 shows a front view and a side view of an indwelling device of a seventh embodiment;
Fig. 18 shows a side sectional view of the indwelling device of the seventh embodiment;
Fig. 19 shows a side sectional view of an indwelling device of an eighth embodiment;
Fig. 20 shows a side sectional view of an indwelling device of a ninth embodiment;
Fig. 21 shows a view at a first stage for explaining a step of placing the indwelling device of the eighth embodiment in the body of a patient;
Fig. 22 shows a view at a second stage for explaining a step of placing the indwelling device of the eighth embodiment in the body of the patient;
Fig. 23 shows a view at a third stage for explaining a step of placing the indwelling device of the eighth embodiment in the body of the patient;
Fig. 24 shows a view at a fourth stage for explaining a step of placing the indwelling device of the eighth embodiment in the body of the patient; and
Fig. 25 shows a view at a final stage for explaining a step of placing the indwelling device of the eighth embodiment in the body of the patient.

### Description of Embodiments

Embodiments of the present invention will be described below by referring to the drawings.

### (First Embodiment)

A first embodiment of the present invention will be described first.

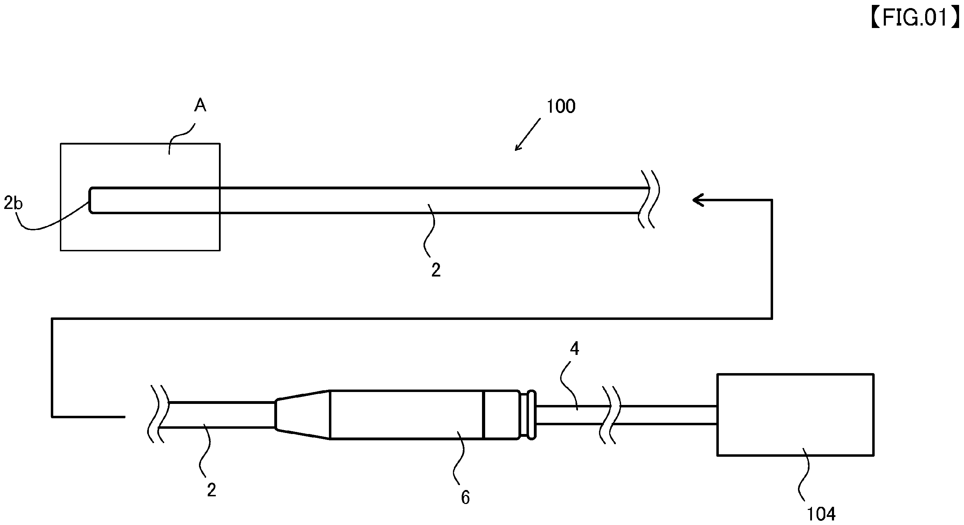

Fig. 1 shows an overall schematic view of a medical instrument for placement of an indwelling device of the present invention in a body. Fig. 2 shows a front view and a side view of an indwelling device of the first embodiment. Fig. 3 shows a side sectional view of the indwelling device of the first embodiment.

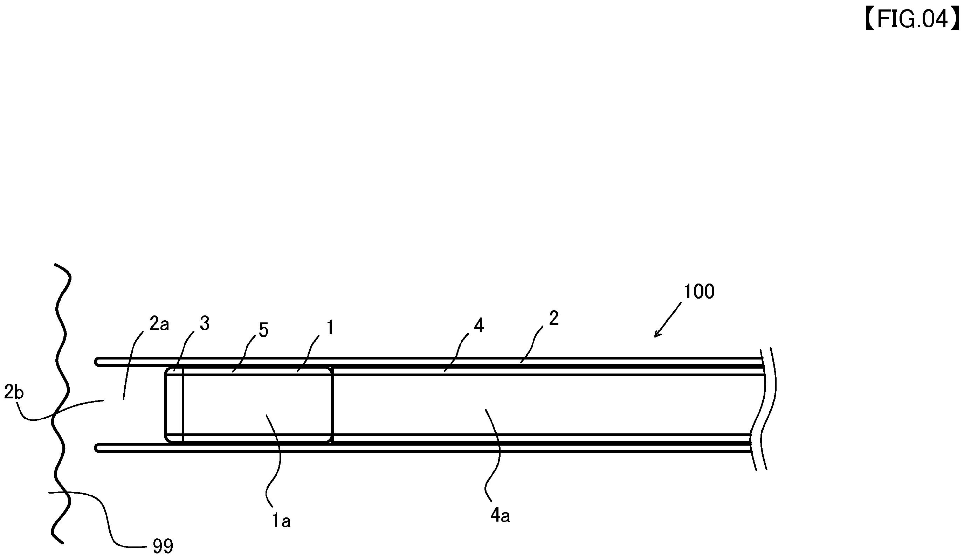

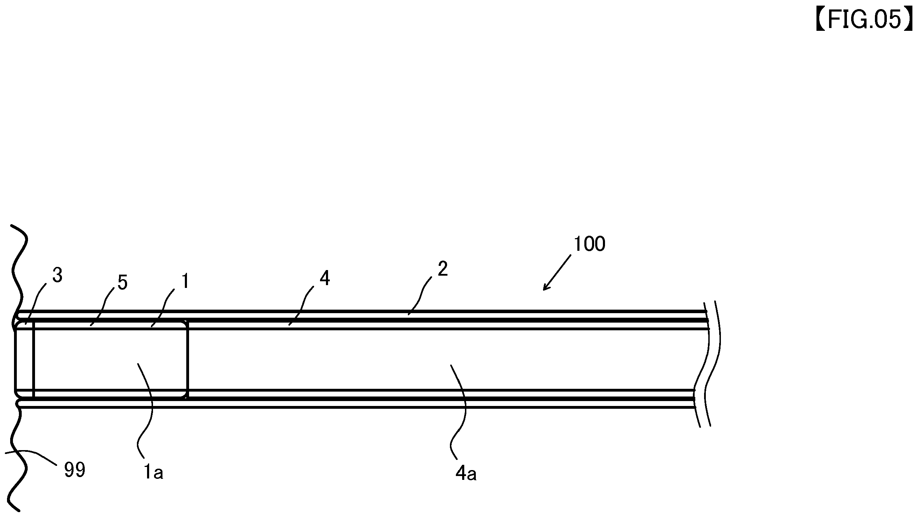

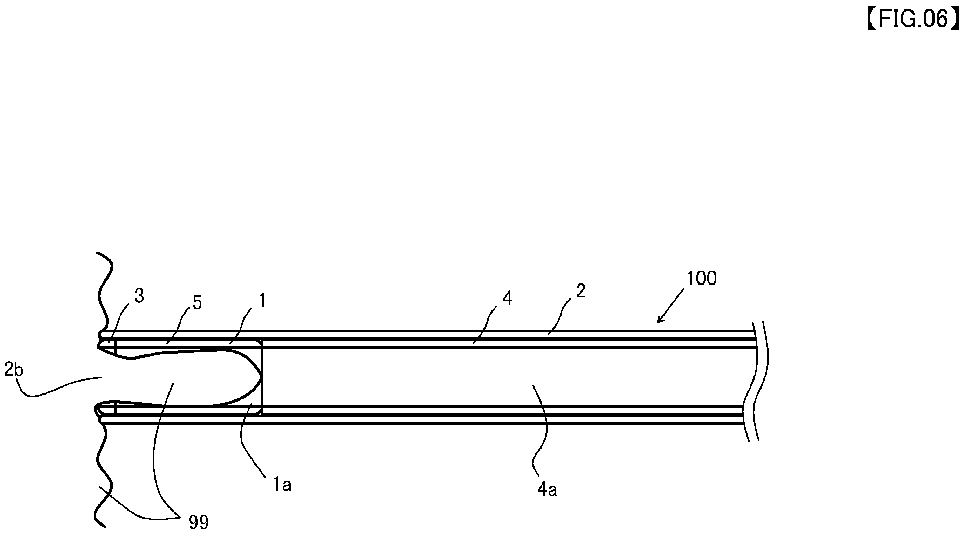

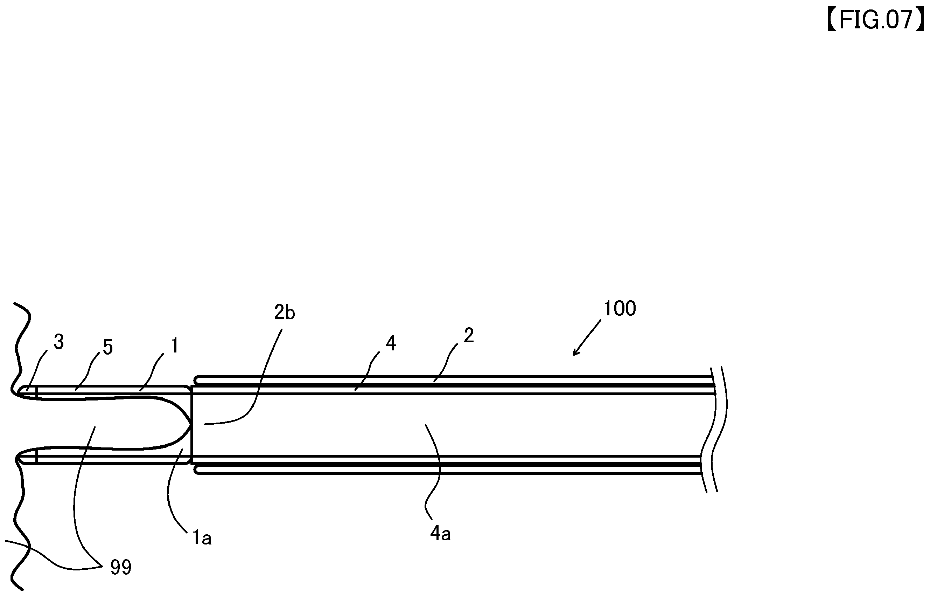

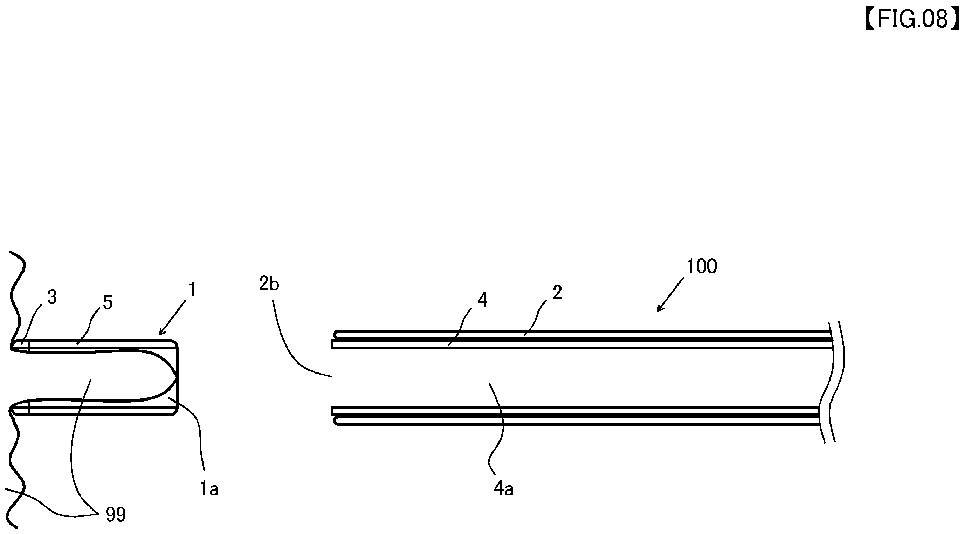

Fig. 4 shows an enlarged view of a section at an area A in Fig. 1 and shows a view at a first stage for explaining a step of placing the indwelling device of the first embodiment in the body of a patient. Fig. 5 shows a view at a second stage. Fig. 6 shows a view at a third stage. Fig. 7 shows a view at a fourth stage. Fig. 8 shows a view at a final stage.

As shown in Fig. 1, a medical instrument 100 for placement of an indwelling device 1 of this embodiment described later (see Figs. 2 and 3) in the body of a patient includes a catheter body 2, a connector 6 connected to the base end of the catheter body 2, a pusher member 4 inserted in the connector 6, and an aspirator 104 connected through the pusher member 4.

The catheter body 2 is a hollow cylindrical and flexible elongated member, and includes a catheter lumen 2a inside the catheter body 2 (see Fig. 4) and an opening portion 2b at the tip of the catheter body 2.

A resin material made of a biocompatible material is used for forming the catheter body 2. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

The connector 6 is a hollow member with a lumen communicating with the catheter lumen 2a of the catheter body 2.

A material for the connector 6 is not particularly limited as long as it has higher rigidity than the material for the catheter body 2. For example, the connector 6 can be made of polyethylene, polypropylene, polycarbonate, polyacetal, polyvinyl chloride (PVC), polybutadiene (PBD), or ABS resin. The material used in this embodiment is polyvinyl chloride (PVC).

The aspirator 104 is a unit connected through the pusher member 4. Another member such as a tube may be interposed between the pusher member 4 and the aspirator 104. The aspirator 104 is actuated for aspiration of a lesion on an affected part of a patient into the indwelling device of the present invention described later. The aspirator 104 is switched on and off by manipulator's operation.

The pusher member 4 can be inserted into the catheter lumen 2a of the catheter body 2. Like the catheter body 2, the pusher member 4 is a hollow cylindrical and flexible elongated member including a lumen 4a inside the pusher member 4 (see Fig. 4).

The pusher member 4 of this embodiment has a hollow cylindrical shape conforming to the shape of the indwelling device of the present invention described later. However, the shape of the pusher member 4 is not limited to a hollow cylinder as long as it allows push-in of the indwelling device of the present invention.

The pusher member 4 is a member for pushing the indwelling device of the present invention described later against an affected part of a patient and then separating the indwelling device from the catheter body 2. The indwelling device and the pusher member 4 of the present invention are inserted from the base end of the connector 6. The tips of the inserted indwelling device and pusher member 4 pass through the lumen of the connector 6 and the catheter lumen 2a of the catheter body 2 to reach the vicinity of a tip portion of the catheter body 2.

As shown in Figs. 2 and 3, the indwelling device 1 of this embodiment is a hollow tubular device with a lumen 1a inside the indwelling device 1. More specifically, the indwelling device 1 has a hollow cylindrical shape. The indwelling device 1 includes a device body 5, and a device tip portion 3 connected to the tip of the device body 5.

The indwelling device 1 is made of soft resin as a whole. In particular, the device tip portion 3 is made of an elastic member.

A resin material made of a biocompatible material is used for forming the device body 5. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

An elastic resin material made of a biocompatible material is used for forming the device tip portion 3. Various types of materials are usable such as polyurethane elastomer, nylon 12 elastomer, silicone rubber, and fluoro-rubber. At present, polyurethane elastomer is the most functionally excellent material particularly for the device tip portion 3.

Steps of placing the indwelling device 1 of the present invention in the body of a patient using the medical instrument 100 will be described by referring to the drawings.

### (First Stage)

First, the indwelling device 1 is inserted into the catheter lumen 2a of the catheter body 2 of the medical instrument 100. Then, the pusher member 4 is inserted while in abutting contact with the base end of the indwelling device 1. While the indwelling device 1 is pushed with the pusher member 4, the indwelling device 1 and the pusher member 4 are advanced to the vicinity of the tip of the catheter body 2. In this state, the tip of the catheter body 2 is advanced to the vicinity of an affected part 99 of the patient (see Fig. 4).

### (Second Stage)

After the tip of the catheter body 2 is brought into abutting contact with the affected part 99 of the patient, the indwelling device 1 in the catheter lumen 2a is pushed with the pusher member 4 to bring the tip of the indwelling device 1 into abutting contact with the affected part 99 of the patient (see Fig. 5).

### (Third Stage)

Next, the aspirator 104 is actuated to generate negative pressure in the lumen 4a of the pusher member 4 and in the lumen 1a of the indwelling device 1, thereby aspirating a lesion on the affected part 99 through the tip of the indwelling device 1 into the lumen 1a of the indwelling device 1 (see Fig. 6).

### (Fourth Stage)

After the lesion on the affected part 99 is aspirated into the lumen 1a of the indwelling device 1, the catheter body 2 is pulled with the pusher member 4 in a grasped state, thereby exposing the indwelling device 1 from the opening portion 2b of the catheter body 2 (see Fig. 7). At this time, as the device tip portion 3 of the indwelling device 1 is made of an elastic member, the affected part 99 is compressed with the device tip portion 3 so that the indwelling device 1 does not come off the affected part 99.

### (Final Stage)

After the indwelling device 1 is exposed from the opening portion 2b of the catheter body 2, the catheter body 2 and the pusher member 4 are separated from the indwelling device 1. Then, the steps are finished (see Fig. 8).

The indwelling device 1 of this embodiment is configured with a hollow tubular body, and the device tip portion 3 corresponding to a tip portion of the hollow tubular body is made of an elastic member. Thus, after the tip portion of the indwelling device 1 is brought into abutting contact with a lesion and the lesion is aspirated from a different end of the indwelling device 1 into the lumen 1a of the indwelling device 1, the indwelling device 1 can reliably be placed in a body using the elastic force of the device tip portion 3 and the lesion can reliably be necrotized using the elastic force of the device tip portion 3.

### (Second Embodiment)

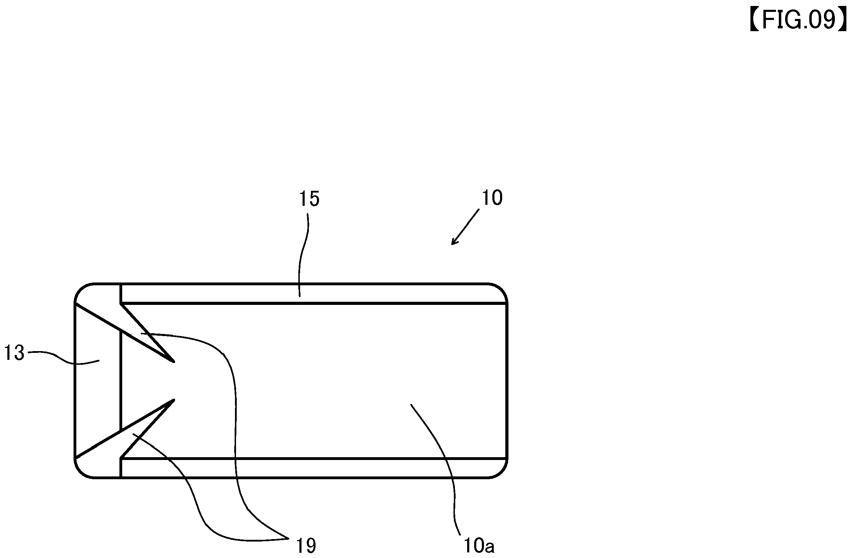

A second embodiment of the present invention will be described next. Fig. 9 shows a side sectional view of an indwelling device of the second embodiment.

An indwelling device 10 of this embodiment differs from the indwelling device 1 of the first embodiment in that the indwelling device 10 of this embodiment is provided with a check valve at a tip portion of the indwelling device 10, unlike the indwelling device 1 without a check valve.

It is noted that the indwelling device 10 of this embodiment can also be placed in the body of a patient using the medical instrument 100 shown in Fig. 1.

As shown in Fig. 9, the indwelling device 10 of this embodiment is a hollow tubular device with a lumen 10a inside the indwelling device 10. More specifically, the indwelling device 10 has a hollow cylindrical shape. The indwelling device 10 includes a device body 15, a device tip portion 13 connected to the tip of the device body 15, and a check valve 19 extending from the device tip portion 13 toward the base end (toward the right of the drawing) and toward the center in a section while having a shape tapered toward the tip in a section.

The check valve 19 is provided to prevent leakage of a lesion to the outside after the lesion is once aspirated into the indwelling device 10. The form of the check valve 19 is not particularly limited. For example, one check valve having a mortal shape may be provided so as to extend over an entire circumference in the cross section of the indwelling device 10. Alternatively, the circumference in the cross section of the indwelling device 10 may be divided, and multiple check valves may be formed separately along the entire circumference in the cross section of the indwelling device 10.

It is noted that description of steps of placing the indwelling device 10 of the present invention in the body of a patient using the medical instrument 100 will be omitted as these steps are the same as those of the first embodiment.

The indwelling device 10 of this embodiment is configured with a hollow tubular body, and the device tip portion 13 corresponding to a tip portion of the hollow tubular body is made of an elastic member. Thus, after the device tip portion 13 of the indwelling device 10 is brought into abutting contact with a lesion and the lesion is aspirated from the base end of the indwelling device 10 into the lumen 10a of the indwelling device 10, the indwelling device 10 can reliably be placed in a body using the elastic force of the device tip portion 13 and the lesion can reliably be necrotized using the elastic force of the device tip portion 13.

Further, in the indwelling device 10 of this embodiment, the check valve 19 is provided at the device tip portion 13. This prevents leakage of a lesion to the outside of the indwelling device 10 to allow the lesion to be held reliably inside the indwelling device 10.

### (Third Embodiment)

A third embodiment of the present invention will be described next.

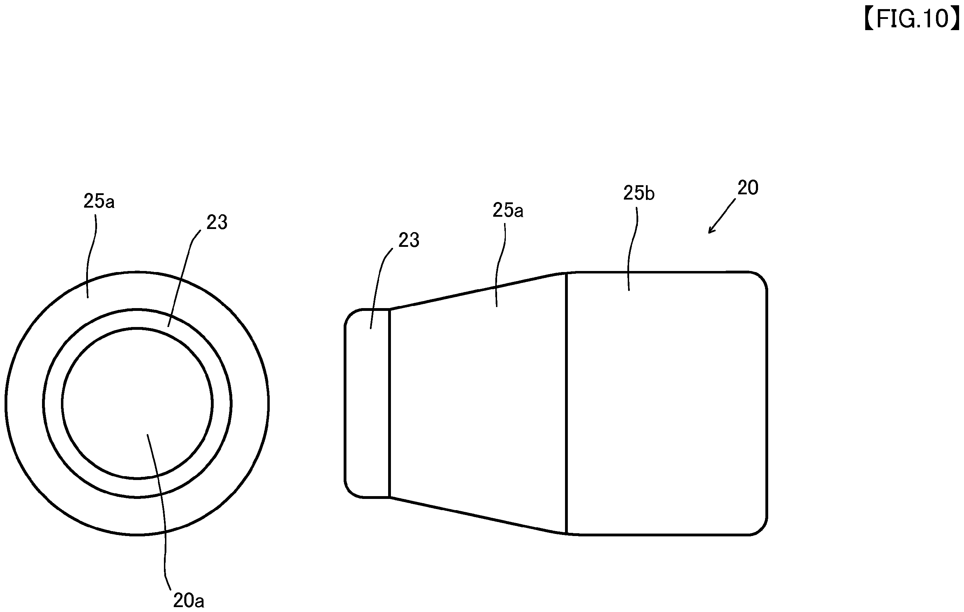

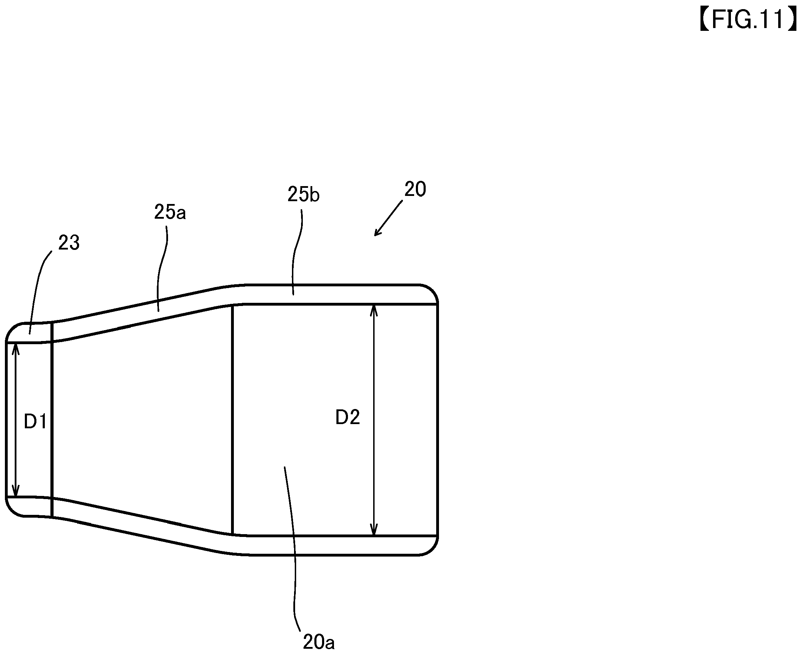

Fig. 10 shows a front view and a side view of an indwelling device of the third embodiment. Fig. 11 shows a side sectional view of the indwelling device of the third embodiment.

As shown in Figs. 10 and 11, an indwelling device 20 of this embodiment is a hollow tubular device with a lumen 20a inside the indwelling device 20. The indwelling device 20 includes a device body cylindrical portion 25b having an inner diameter of D2, a device body tapered portion 25a having a tapered truncated conical shape formed at the tip of the device body cylindrical portion 25b, and a device tip portion 23 connected to the tip of the device body tapered portion 25a and having an inner diameter of D1 smaller than D2.

The device body cylindrical portion 25b and the device body tapered portion 25a correspond to a "diameter-increased portion" of the present invention.

The indwelling device 20 of this embodiment can also be placed in the body of a patient using the medical instrument 100 shown in Fig. 1.

Like the indwelling device 1 of the first embodiment, the indwelling device 20 is made of soft resin as a whole. In particular, the device tip portion 23 is made of an elastic member.

A resin material made of a biocompatible material is used for forming the device body tapered portion 25a and the device body cylindrical portion 25b. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

An elastic resin material made of a biocompatible material is used for forming the device tip portion 23. Various types of resin materials are usable such as polyurethane elastomer, nylon 12 elastomer, silicone rubber, and fluoro-rubber, for example. At present, polyurethane elastomer is the most functionally excellent material particularly for the device tip portion 23 of the indwelling device 20.

The indwelling device 20 of this embodiment is configured with a hollow tubular body, and the device tip portion 23 corresponding to a tip portion of the hollow tubular body is made of an elastic member. Thus, after the device tip portion 23 of the indwelling device 20 is brought into abutting contact with a lesion and the lesion is aspirated from the base end of the indwelling device 20 into the lumen 20a of the indwelling device 20, the indwelling device 20 can reliably be placed in a body using the elastic force of the device tip portion 23 and the lesion can reliably be necrotized using the elastic force of the device tip portion 23.

Further, the indwelling device 20 of this embodiment includes the device body tapered portion 25a and the device body cylindrical portion 25b (corresponding to the "diameter-increased portion" of the present invention) provided closer to the base end than the device tip portion 23 and having the inner diameter larger than the inner diameter D1 of the device tip portion 23. Thus, even in the case of a lesion having a large volume, the indwelling device 20 can be placed in a body reliably using the elastic force of the device tip portion 23 and the lesion can be necrotized reliably using the elastic force of the device tip portion 23.

### (Fourth Embodiment)

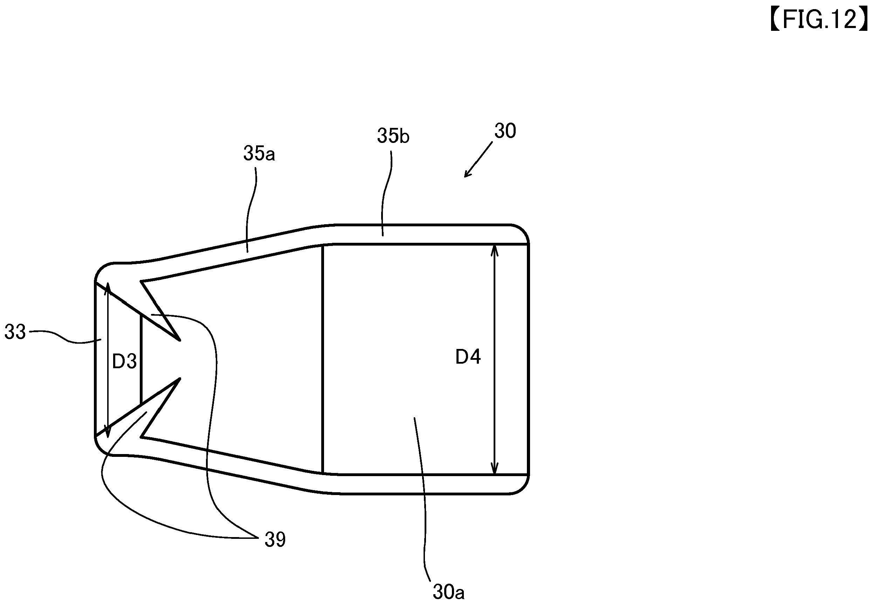

A fourth embodiment of the present invention will be described next. Fig. 12 shows a side sectional view of an indwelling device of the fourth embodiment.

An indwelling device 30 of this embodiment differs from the indwelling device 20 of the third embodiment in that the indwelling device 30 of this embodiment is provided with a check valve 39 at a tip portion of the indwelling device 30, unlike the indwelling device 20 without a check valve.

As shown in Fig. 12, the indwelling device 30 of this embodiment is a hollow tubular device with a lumen 30a inside the indwelling device 30. The indwelling device 30 includes a device body cylindrical portion 35b having an inner diameter of D4, a device body tapered portion 35a having a tapered truncated conical shape formed at the tip of the device body cylindrical portion 35b, a device tip portion 33 connected to the tip of the device body tapered portion 35a and having an inner diameter of D3 smaller than D4, and the check valve 39 provided at the device tip portion 33, extending toward the base end (toward the right of the drawing) and toward the center in a section, and having a shape tapered toward the tip in a section.

Like the check valve 19 of the second embodiment, the check valve 39 is provided to prevent leakage of a lesion to the outside after the lesion is once aspirated into the indwelling device 30. The form of the check valve 39 is not particularly limited. For example, one check valve having a mortal shape may be provided so as to extend over an entire circumference in the cross section of the indwelling device 30. Alternatively, the circumference in the cross section of the indwelling device 30 may be divided, and multiple check valves may be formed separately along the entire circumference in the cross section of the indwelling device 30.

It is noted that the device body cylindrical portion 35b and the device body tapered portion 35a correspond to the "diameter-increased portion" of the present invention.

The indwelling device 30 of this embodiment can also be placed in the body of a patient using the medical instrument 100 shown in Fig. 1.

The indwelling device 30 of this embodiment is configured with a hollow tubular body, and the device tip portion 33 corresponding to a tip portion of the hollow tubular body is made of an elastic member. Thus, after the device tip portion 33 of the indwelling device 30 is brought into abutting contact with a lesion and the lesion is aspirated from the base end of the indwelling device 30 into the lumen 30a of the indwelling device 30, the indwelling device 30 can reliably be placed in a body using the elastic force of the device tip portion 33 and the lesion can reliably be necrotized using the elastic force of the device tip portion 33.

Further, the indwelling device 30 of this embodiment includes the device body tapered portion 35a and the device body cylindrical portion 35b (corresponding to the "diameter-increased portion" of the present invention) provided closer to the base end than the device tip portion 33 and having the inner diameter larger than the inner diameter of the device tip portion 33. Thus, even in the case of a lesion having a large volume, the indwelling device 30 can be placed in a body reliably using the elastic force of the device tip portion 33 and the lesion can be necrotized reliably using the elastic force of the device tip portion 33.

Further, the indwelling device 30 of this embodiment is provided with the check valve 39 at the device tip portion 33. This prevents leakage of a lesion to the outside of the indwelling device 30 to allow the lesion to be held reliably inside the indwelling device 30.

### (Fifth Embodiment)

A fifth embodiment of the present invention will be described next.

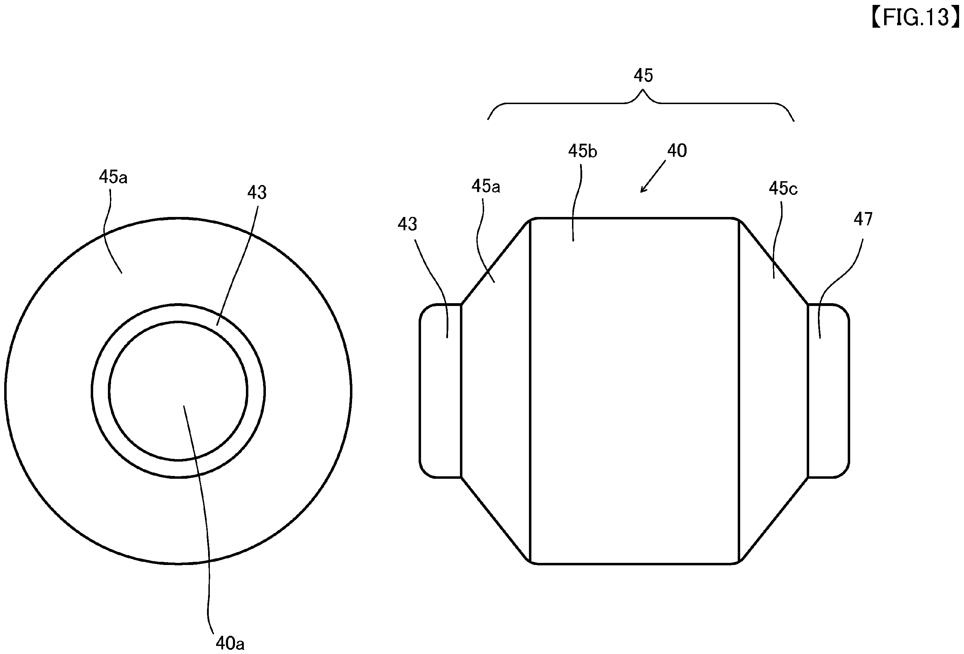

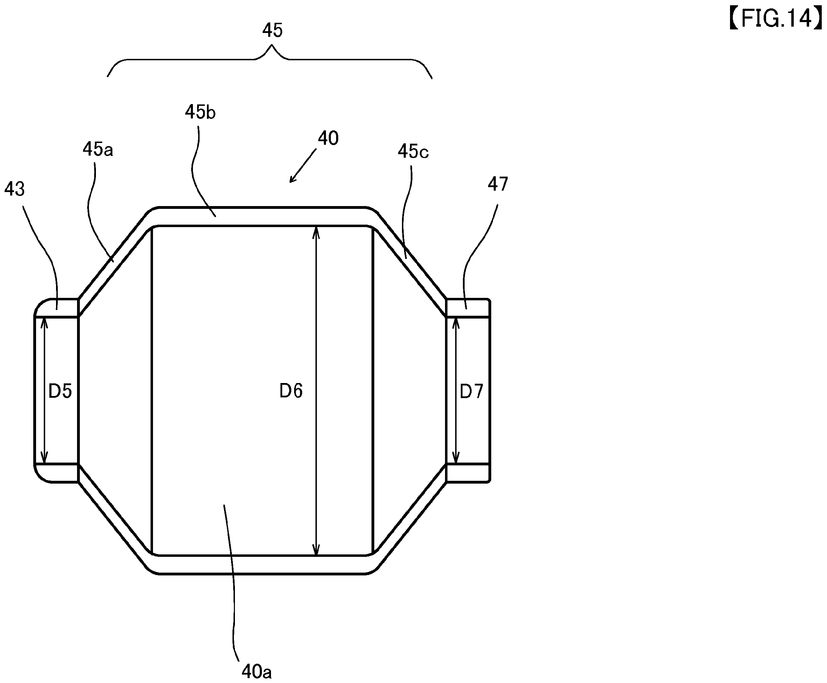

Fig. 13 shows a front view and a side view of an indwelling device of the fifth embodiment. Fig. 14 shows a side sectional view of the indwelling device of the fifth embodiment.

As shown in Figs. 13 and 14, an indwelling device 40 of this embodiment is a hollow tubular device with a lumen 40a inside the indwelling device 40.

The indwelling device 40 includes a device tip portion 43 having an inner diameter of D5, a device body first tapered portion 45a having a reverse tapered truncated conical shape connected to the base end of the device tip portion 43, a device body cylindrical portion 45b formed at the base end of the device body first tapered portion 45a and having an inner diameter of D6 larger than D5, a device body second tapered portion 45c having a tapered truncated conical shape formed at the base end of the device body cylindrical portion 45b, and a device base end portion 47 connected to the base end of the device body second tapered portion 45c and having an inner diameter of D7 smaller than D6.

It is noted that the device body first tapered portion 45a, the device body cylindrical portion 45b, and the device body second tapered portion 45c correspond to the "diameter-increased portion" of the present invention.

The device base end portion 47 corresponds to a "diameter-reduced portion" of the present invention.

It is noted that in this embodiment, the inner diameter D5 and the inner diameter D7 are described as being equal. Meanwhile, both the inner diameter D5 and the inner diameter D7 are only required to be smaller than the inner diameter D6 and are not always required to be equal.

The indwelling device 40 of this embodiment can be placed in the body of a patient using the medical instrument 100 shown in Fig. 1.

Like the indwelling device 1 of the first embodiment, the indwelling device 40 is made of soft resin as a whole. In particular, the device tip portion 43 and the device base end portion 47 are made of an elastic member.

A resin material made of a biocompatible material is used for forming the device body first tapered portion 45a, the device body cylindrical portion 45b, and the device body second tapered portion 45c. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

An elastic resin material made of a biocompatible material is used for forming the device tip portion 43 and the device base end portion 47. Various types of materials are usable such as polyurethane elastomer, nylon 12 elastomer, silicone rubber, and fluoro-rubber. At present, polyurethane elastomer is the most functionally excellent material particularly for the device tip portion 43 and the device base end portion 47 of the indwelling device 40.

It is noted that the reason for using the elastic member as a material for forming both the device tip portion 43 and the device base end portion 47 is to allow insertion from either end portion of the indwelling device 40 when the indwelling device 40 is to be inserted into the catheter body 2.

The indwelling device 40 of this embodiment is configured with a hollow tubular body, and the device tip portion 43 corresponding to a tip portion of the hollow tubular body is made of an elastic member. Thus, after the device tip portion 43 of the indwelling device 40 is brought into abutting contact with a lesion and the lesion is aspirated from the base end of the indwelling device 40 into the lumen 40a of the indwelling device 40, the indwelling device 40 can reliably be placed in a body using the elastic force of the device tip portion 43 and the lesion can reliably be necrotized using the elastic force of the device tip portion 43.

The indwelling device 40 of this embodiment includes the device tip portion 43, the device body first tapered portion 45a having a reverse tapered truncated conical shape connected to the base end of the device tip portion 43, the device body cylindrical portion 45b formed at the base end of the device body first tapered portion 45a, the device body second tapered portion 45c having a tapered truncated conical shape formed at the base end of the device body cylindrical portion 45b, and the device base end portion 47 connected to the base end of the device body second tapered portion 45c. Thus, even in the case of a lesion having a large volume, the indwelling device 40 can be placed in a body reliably using the elastic force of the device tip portion 43 and the lesion can be necrotized reliably using the elastic force of the device tip portion 43. Further, the lesion can reliably be held inside the indwelling device 40.

### (Sixth Embodiment)

A sixth embodiment of the present invention will be described next.

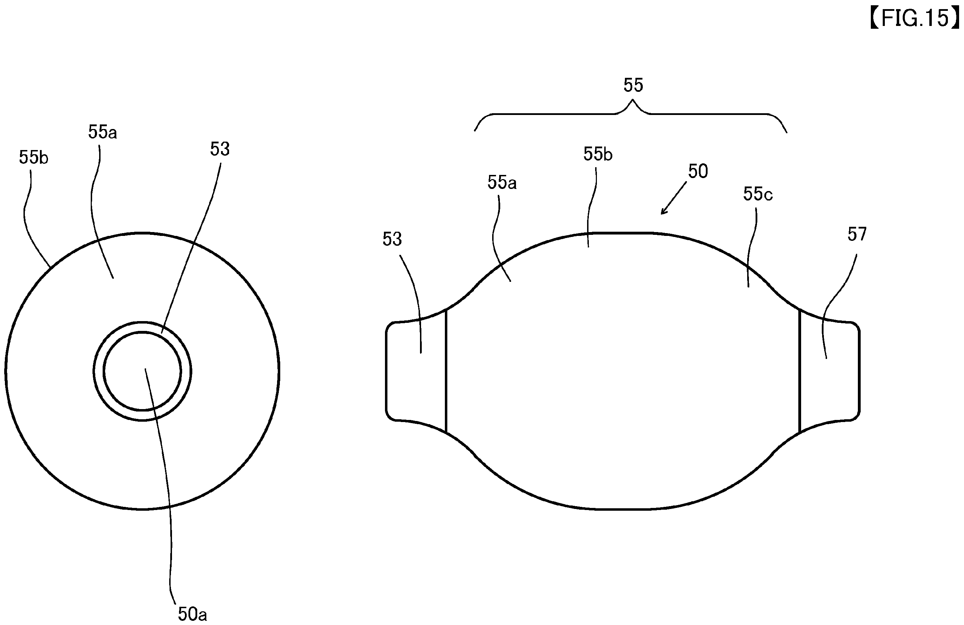

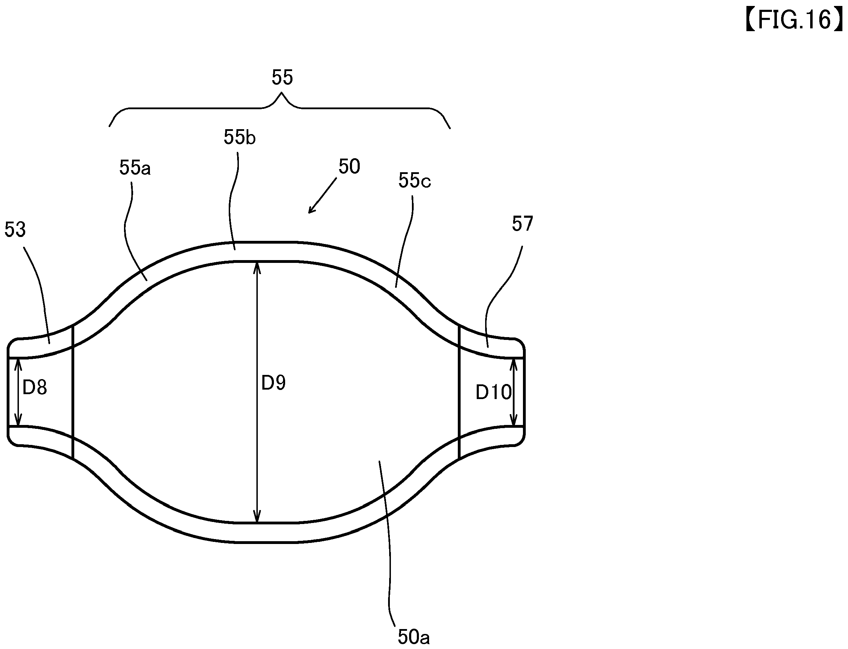

Fig. 15 shows a front view and a side view of an indwelling device of the sixth embodiment. Fig. 16 shows a side sectional view of the indwelling device of the sixth embodiment.

As shown in Figs. 15 and 16, an indwelling device 50 of this embodiment is a hollow tubular device with a lumen 50a inside the indwelling device 50.

The indwelling device 50 includes a device tip portion 53 having an inner diameter of D8, a device body first streamlined portion 55a having a reverse tapered shape connected to the base end of the device tip portion 53, a device body cylindrical portion 55b formed at the base end of the device body first streamlined portion 55a and having an inner diameter of D9 larger than D8, a device body second streamlined portion 55c having a tapered shape formed at the base end of the device body cylindrical portion 55b, and a device base end portion 57 connected to the base end of the device body second streamlined portion 55c and having an inner diameter of D10 smaller than D9.

It is note that the device body first streamlined portion 55a, the device body cylindrical portion 55b, and the device body second streamlined portion 55c correspond to the "diameter-increased portion" of the present invention.

The device base end portion 57 corresponds to the "diameter-reduced portion" of the present invention.

In this embodiment, the inner diameter D8 and the inner diameter D10 are described as being equal. Meanwhile, both the inner diameter D8 and the inner diameter D10 are only required to be smaller than the inner diameter D9 and are not always required to be equal.

The indwelling device 50 of this embodiment can be placed in the body of a patient using the medical instrument 100 shown in Fig. 1.

Like the indwelling device 1 of the first embodiment, the indwelling device 50 is made of soft resin as a whole. In particular, the device tip portion 53 and the device base end portion 57 are made of an elastic member.

A resin material made of a biocompatible material is used for forming the device body first streamlined portion 55a, the device body cylindrical portion 55b, and the device body second streamlined portion 55c. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

An elastic resin material made of a biocompatible material is used for forming the device tip portion 53 and the device base end portion 57. Various types of materials are usable such as polyurethane elastomer, nylon 12 elastomer, silicone rubber, and fluoro-rubber. At present, polyurethane elastomer is the most functionally excellent material particularly for the device tip portion 53 and the device base end portion 57 of the indwelling device 50.

It is noted that the reason for using the elastic member as a material for forming both the device tip portion 53 and the device base end portion 57 is to allow insertion from either end portion of the indwelling device 50 when the indwelling device 50 is to be inserted into the catheter body 2.

The indwelling device 50 of this embodiment is configured with a hollow tubular body, and the device tip portion 53 corresponding to a tip portion of the hollow tubular body is made of an elastic member. Thus, after the device tip portion 53 of the indwelling device 50 is brought into abutting contact with a lesion and the lesion is aspirated from the base end of the indwelling device 50 into the lumen 50a of the indwelling device 50, the indwelling device 50 can reliably be placed in a body using the elastic force of the device tip portion 53 and the lesion can reliably be necrotized using the elastic force of the device tip portion 53.

The indwelling device 50 of this embodiment includes the device tip portion 53, the device body first streamlined portion 55a having a reverse tapered shape connected to the base end of the device tip portion 53, the device body cylindrical portion 55b formed at the base end of the device body first streamlined portion 55a, the device body second streamlined portion 55c having a tapered shape formed at the base end of the device body cylindrical portion 55b, and the device base end portion 57 connected to the base end of the device body second streamlined portion 55c. Thus, even in the case of a lesion having a large volume, the indwelling device 50 can be placed in a body reliably using the elastic force of the device tip portion 53 and the lesion can be necrotized reliably using the elastic force of the device tip portion 53. Further, the lesion can reliably be held inside the indwelling device 50. Additionally, the indwelling device 50 can easily be inserted into a catheter, for example, for guiding the indwelling device 50 into the body.

### (Seventh Embodiment)

A seventh embodiment of the present invention will be described next.

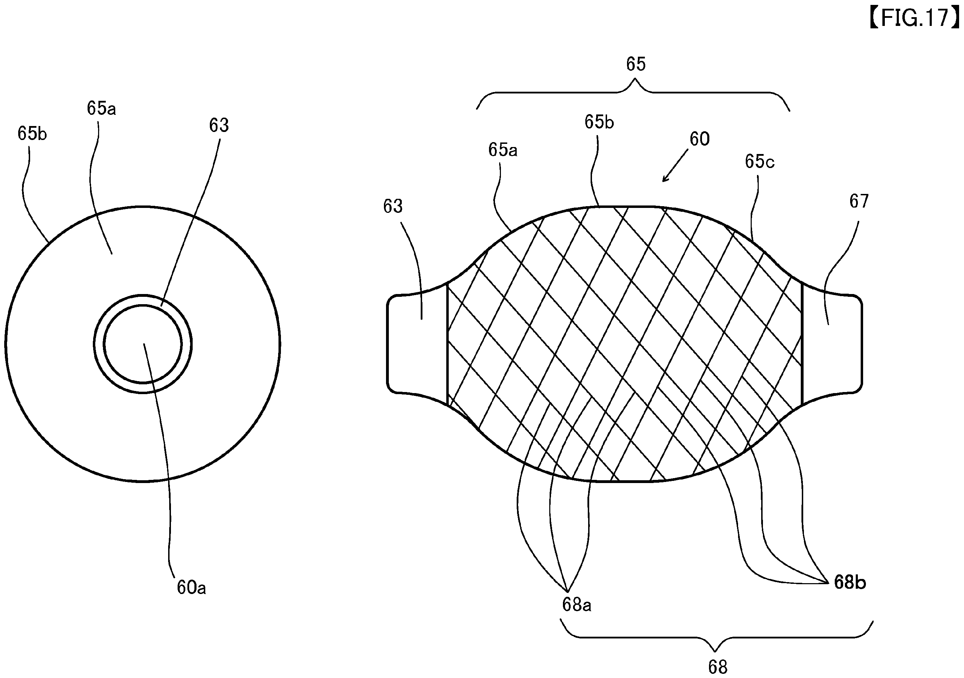

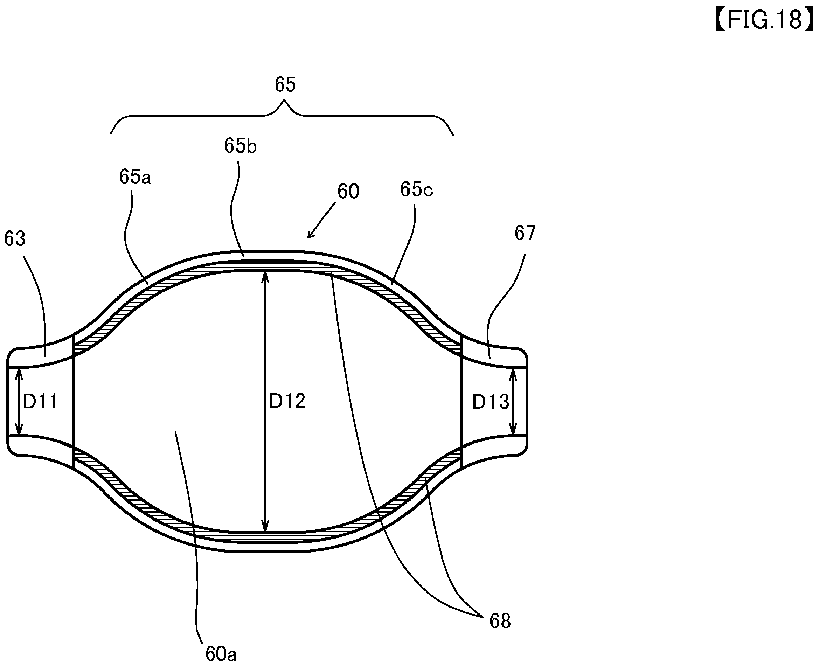

Fig. 17 shows a front view and a side view of an indwelling device of the seventh embodiment. Fig. 18 shows a side sectional view of the indwelling device of the seventh embodiment.

As shown in Figs. 17 and 18, an indwelling device 60 of this embodiment is a hollow tubular device with a lumen 60a inside the indwelling device 60.

The indwelling device 60 includes a device tip portion 63 having an inner diameter of D11, a device body first streamlined portion 65a having a reverse tapered shape connected to the base end of the device tip portion 63, a device body cylindrical portion 65b formed at the base end of the device body first streamlined portion 65a and having an inner diameter of D12 larger than D11, a device body second streamlined portion 65c having a tapered shape formed at the base end of the device body cylindrical portion 65b, and a device base end portion 67 connected to the base end of the device body second streamlined portion 65c and having an inner diameter of D13 smaller than D12.

The device body first streamlined portion 65a, the device body cylindrical portion 65b, and the device body second streamlined portion 65c each have a two-layered structure with an inner layer and an outer layer made of resin covering the inner layer. The inner layer is provided with a braid (braided structure) 68. The braid 68 is configured with multiple element wires 68a wound in one direction and multiple element wires 68b wound in a direction crossing the one direction.

The device body first streamlined portion 65a, the device body cylindrical portion 65b, and the device body second streamlined portion 65c correspond to the "diameter-increased portion" (diameter-increased portion 65) of the present invention.

The device base end portion 67 corresponds to the "diameter-reduced portion" of the present invention.

In this embodiment, the inner diameter D11 and the inner diameter D13 are described as being equal. Meanwhile, both the inner diameter D11 and the inner diameter D13 are only required to be smaller than the inner diameter D12 and are not always required to be equal.

The indwelling device 60 of this embodiment can also be placed in the body of a patient using the medical instrument 100 shown in Fig. 1.

The indwelling device 60 is made of soft resin as a whole. In particular, the device tip portion 63 and the device base end portion 67 are made of an elastic member.

A resin material made of a biocompatible material is used for forming the outer layer of each of the device body first streamlined portion 65a, the device body cylindrical portion 65b, and the device body second streamlined portion 65c. Various types of composite resin materials are usable such as polyamide resin, polyamide elastomer, polyester, polyurethane, polyvinyl chloride resin, polyethylene, polypropylene, polyolefin resin such as ethylene-propylene copolymer, fluorine resin such as Teflon (registered trademark), and ethylene-vinyl acetate copolymer, for example.

An elastic resin material made of a biocompatible material is used for forming the device tip portion 63 and the device base end portion 67. Various types of materials are usable such as polyurethane elastomer, nylon 12 elastomer, silicone rubber, and fluoro-rubber. At present, polyurethane elastomer is the most functionally excellent material particularly for the device tip portion 63 and the device base end portion 67 of the indwelling device 60.

It is noted that the reason for using the elastic member as a material for forming both the device tip portion 63 and the device base end portion 67 is to allow insertion from either end portion of the indwelling device 60 when the indwelling device 60 is to be inserted into the catheter body 2.

A material for the braid 68 forming the inner layer of each of the device body first streamlined portion 65a, the device body cylindrical portion 65b, and the device body second streamlined portion 65c is not particularly limited, as long as it is made of a biocompatible material such as stainless steel, tungsten, or Ni-Ti based alloy, for example. Meanwhile, the indwelling device of this embodiment is a self-expanding indwelling device as described later, so that shape-memory alloy is used. More specifically, Ni-Ti based alloy is used.

For visual recognition of the position of the indwelling device by radioscopy, it is desirable that the braid 68 be partially made of a radiopaque material such as platinum alloy or tungsten, for example.

The indwelling device 60 of this embodiment is configured with a hollow tubular body, and the device tip portion 63 corresponding to a tip portion of the hollow tubular body is made of an elastic member. Thus, after the device tip portion 63 of the indwelling device 60 is brought into abutting contact with a lesion and the lesion is aspirated from the base end of the indwelling device 60 into the lumen 60a of the indwelling device 60, the indwelling device 60 can reliably be placed in a body using the elastic force of the device tip portion 63 and the lesion can reliably be necrotized using the elastic force of the device tip portion 63.

The indwelling device 60 of this embodiment includes the device tip portion 63, the device body first streamlined portion 65a having a reverse tapered shape connected to the base end of the device tip portion 63, the device body cylindrical portion 65b formed at the base end of the device body first streamlined portion 65a, the device body second streamlined portion 65c having a tapered shape formed at the base end of the device body cylindrical portion 65b, and the device base end portion 67 connected to the base end of the device body second streamlined portion 65c. Thus, even in the case of a lesion having a large volume, the indwelling device 60 can be placed in a body reliably using the elastic force of the device tip portion 63 and the lesion can be necrotized reliably using the elastic force of the device tip portion 63. Further, the lesion can reliably be held inside the indwelling device 60. Additionally, the indwelling device 60 can easily be inserted into a catheter, for example, for guiding the indwelling device 60 into the body.

In the indwelling device 60 of this embodiment, the braid 68 and the resin portion covering the braid 68 are provided at each of the device body first streamlined portion 65a, the device body cylindrical portion 65b, and the device body second streamlined portion 65c. Thus, even if a lesion in a compressed state is aspirated into the indwelling device 60, the lesion does not break and the lesion can be held reliably inside the indwelling device 60.

### (Eighth Embodiment)

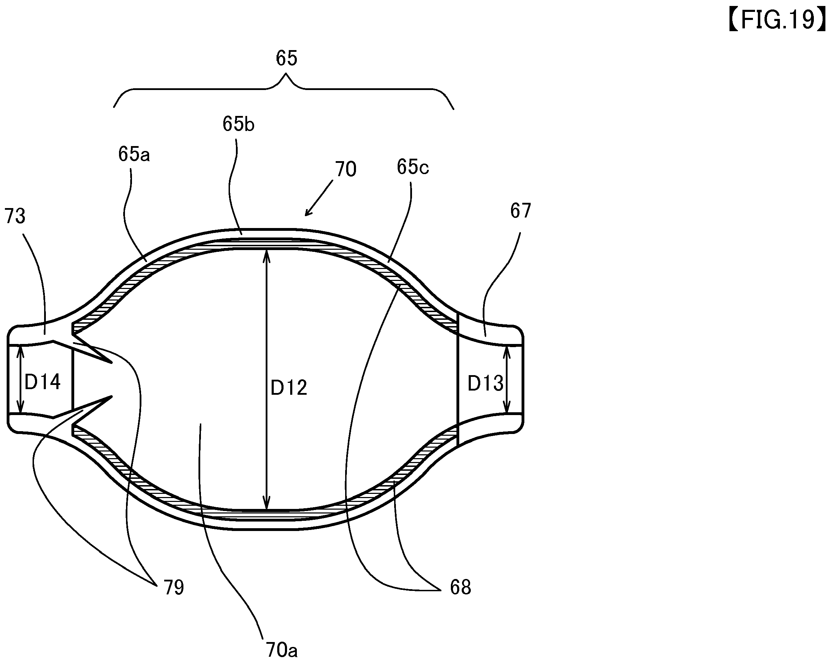

An eighth embodiment of the present invention will be described next. Fig. 19 shows a side sectional view of an indwelling device of the eighth embodiment.

An indwelling device 70 of this embodiment differs from the indwelling device 60 of the seventh embodiment in that the indwelling device 70 of this embodiment is provided with a check valve at a tip portion of the indwelling device 70, unlike the indwelling device 60 without a check valve.

The indwelling device 70 of this embodiment can also be placed in the body of a patient using the medical instrument 100 shown in Fig. 1.

As shown in Fig. 19, the indwelling device 70 of this embodiment is a hollow tubular device with a lumen 70a inside the indwelling device 70.

It is noted that the configuration of the indwelling device 70 is the same as that of the indwelling device 60 except that the indwelling device 70 includes a check valve 79 extending from a device tip portion 73 having an inner diameter of D14 toward the base end (toward the right of the drawing) and toward the center in a section while having a shape tapered toward the tip in a section.

In this embodiment, the inner diameter D 14 and the inner diameter D13 are described as being equal. Meanwhile, both the inner diameter D14 and the inner diameter D13 are only required to be smaller than the inner diameter D12 and are not always required to be equal.

Like the check valve 19 of the second embodiment and the check valve 39 of the fourth embodiment, the check valve 79 is provided to prevent leakage of a lesion to the outside after the lesion is once aspirated into the indwelling device 70. The form of the check valve 79 is not particularly limited. For example, one check valve having a mortal shape may be provided so as to extend over an entire circumference in the cross section of the indwelling device 70. Alternatively, the circumference in the cross section of the indwelling device 70 may be divided, and multiple check valves may be formed separately along the entire circumference in the cross section of the indwelling device 70.

The indwelling device 70 of this embodiment is configured with a hollow tubular body, and the device tip portion 73 corresponding to a tip portion of the hollow tubular body is made of an elastic member. Thus, after the device tip portion 73 of the indwelling device 70 is brought into abutting contact with a lesion and the lesion is aspirated from the base end of the indwelling device 70 into the lumen 70a of the indwelling device 70, the indwelling device 70 can reliably be placed in a body using the elastic force of the device tip portion 73 and the lesion can reliably be necrotized using the elastic force of the device tip portion 73.

The indwelling device 70 of this embodiment includes the device tip portion 73, the device body first streamlined portion 65a having a reverse tapered shape connected to the base end of the device tip portion 73, the device body cylindrical portion 65b formed at the base end of the device body first streamlined portion 65a, the device body second streamlined portion 65c having a tapered shape formed at the base end of the device body cylindrical portion 65b, and the device base end portion 67 connected to the base end of the device body second streamlined portion 65c. Thus, even in the case of a lesion having a large volume, the indwelling device 70 can be placed in a body reliably using the elastic force of the device tip portion 73 and the lesion can be necrotized reliably using the elastic force of the device tip portion 73. Further, the lesion can reliably be held inside the indwelling device 70. Additionally, the indwelling device 70 can easily be inserted into a catheter, for example, for guiding the indwelling device 70 into the body.

In the indwelling device 70 of this embodiment, the braid 68 and the resin portion covering the braid 68 are provided at each of the device body first streamlined portion 65a, the device body cylindrical portion 65b, and the device body second streamlined portion 65c. Thus, even if a lesion in a compressed state is aspirated into the indwelling device 70, the lesion does not break and the lesion can be held reliably inside the indwelling device 70.

Further, in the indwelling device 70 of this embodiment, the check valve 79 is provided at the device tip portion 73. This prevents leakage of a lesion to the outside of the indwelling device 70 to allow the lesion to be held reliably inside the indwelling device 70.

### (Ninth Embodiment)

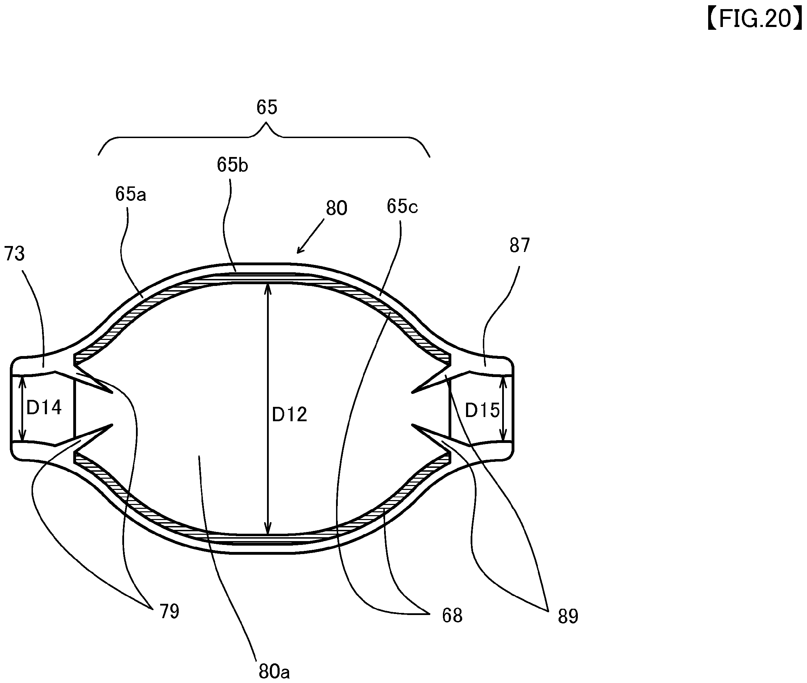

A ninth embodiment of the present invention will be described next. Fig. 20 shows a side sectional view of an indwelling device of the ninth embodiment.

An indwelling device 80 of this embodiment differs from the indwelling device 70 of the eighth embodiment in that the indwelling device 80 of this embodiment is provided with a check valve 89 at a base end portion of the indwelling device 80, unlike the indwelling device 70 without a check valve at a base end portion.

The indwelling device 80 of this embodiment can also be placed in the body of a patient using the medical instrument 100 shown in Fig. 1.

As shown in Fig. 20, the indwelling device 80 of this embodiment is a hollow tubular device with a lumen 80a inside the indwelling device 80.

The configuration of the indwelling device 80 is the same as that of the indwelling device 70 except that the indwelling device 80 includes the check valve 89 extending from a device base end portion 87 having an inner diameter of D15 toward the tip (toward the left of the drawing) and toward the center in a section while having a shape tapered toward the tip in a section.

It is noted that in this embodiment, the inner diameter D14 and the inner diameter D15 are described as being equal. Meanwhile, both the inner diameter D14 and the inner diameter D15 are only required to be smaller than the inner diameter D12 and are not always required to be equal.

Like the check valve 19 of the second embodiment, the check valve 39 of the fourth embodiment, and the check valve 79 of the eighth embodiment, the check valve 89 is provided to prevent leakage of a lesion to the outside after the lesion is once aspirated into the indwelling device 80. The form of the check valve 89 is not particularly limited. For example, one check valve having a mortal shape may be provided at the base end of the indwelling device 80 so as to extend over an entire circumference in the cross section of the indwelling device 80. Alternatively, the circumference in the cross section of the indwelling device 80 may be divided at the base end of the indwelling device 80, and multiple check valves may be formed separately along the entire circumference in the cross section of the indwelling device 80 at the base end of the indwelling device 80.

The indwelling device 80 of this embodiment is configured with a hollow tubular body, and the device tip portion 73 corresponding to a tip portion of the hollow tubular body is made of an elastic member. Thus, after the device tip portion 73 of the indwelling device 80 is brought into abutting contact with a lesion and the lesion is aspirated from the base end of the indwelling device 80 into the lumen 80a of the indwelling device 80, the indwelling device 80 can reliably be placed in a body using the elastic force of the device tip portion 73 and the lesion can reliably be necrotized using the elastic force of the device tip portion 73.

The indwelling device 80 of this embodiment includes the device tip portion 73, the device body first streamlined portion 65a having a reverse tapered shape connected to the base end of the device tip portion 73, the device body cylindrical portion 65b formed at the base end of the device body first streamlined portion 65a, the device body second streamlined portion 65c having a tapered shape formed at the base end of the device body cylindrical portion 65b, and the device base end portion 87 connected to the base end of the device body second streamlined portion 65c. Thus, even in the case of a lesion having a large volume, the indwelling device 80 can be placed in a body reliably using the elastic force of the device tip portion 73 and the lesion can be necrotized reliably using the elastic force of the device tip portion 73. Further, the lesion can reliably be held inside the indwelling device 80. Additionally, the indwelling device 80 can easily be inserted into a catheter, for example, for guiding the indwelling device 80 into the body.

In the indwelling device 80 of this embodiment, the braid 68 and the resin portion covering the braid 68 are provided at each of the device body first streamlined portion 65a, the device body cylindrical portion 65b, and the device body second streamlined portion 65c. Thus, even if a lesion in a compressed state is aspirated into the indwelling device 80, the lesion does not break and the lesion can be held reliably inside the indwelling device 80.

Further, in the indwelling device 80 of this embodiment, the check valve 79 is provided at the device tip portion 73 and the check valve 89 is provided at the device base end portion 87. This further prevents leakage of a lesion to the outside of the indwelling device 80 and allows the lesion to be held reliably inside the indwelling device 80.

Lastly, steps of placing the indwelling device 70 of the present invention in the body of a patient using the medical instrument 100 will be described by referring to the drawings.

It is noted that in these steps, a pusher member 14 with a lumen 14a having a smaller inner diameter than the pusher member 4 is used to allow the indwelling device 70 to be pushed efficiently.

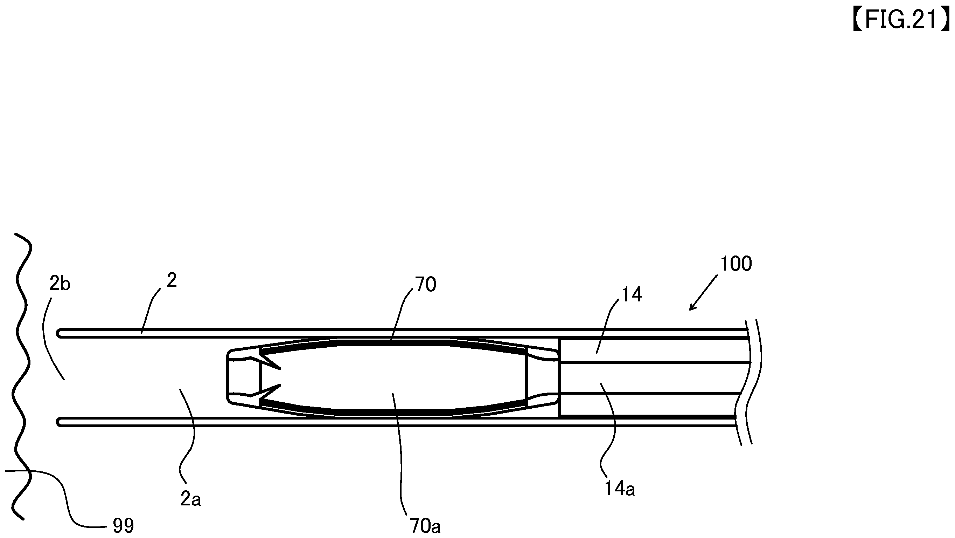

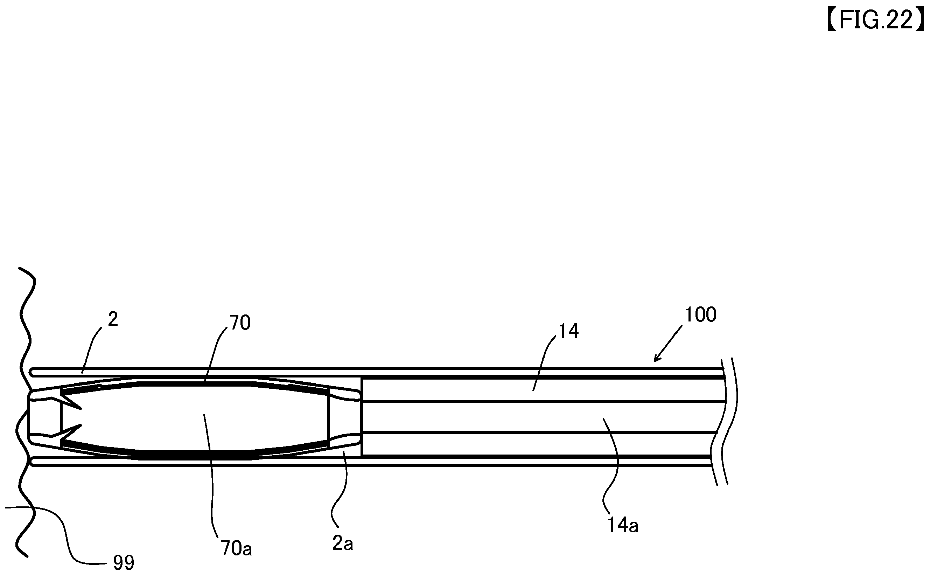

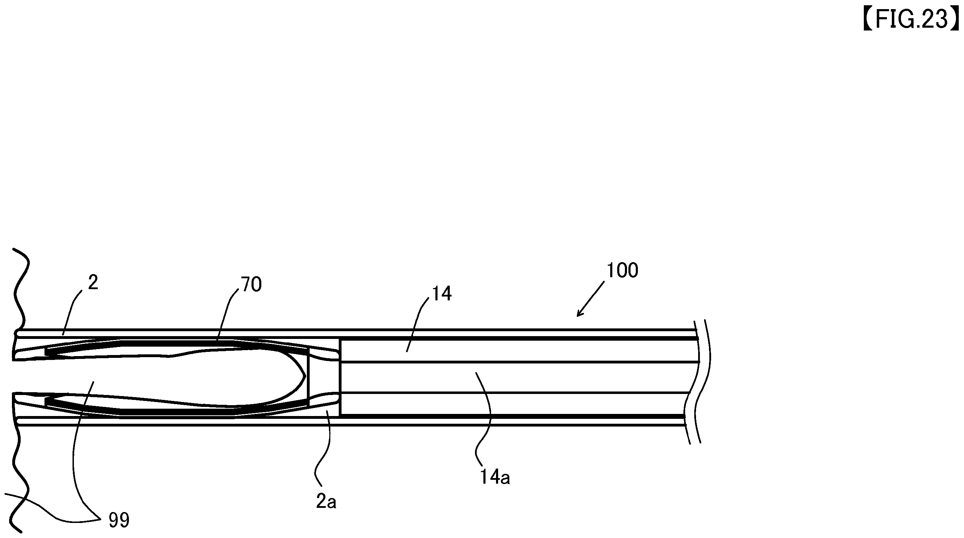

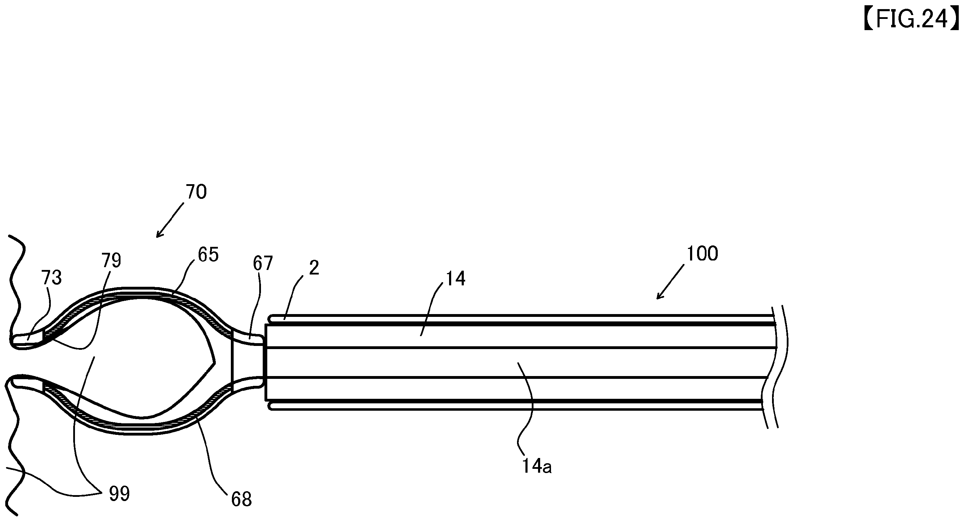

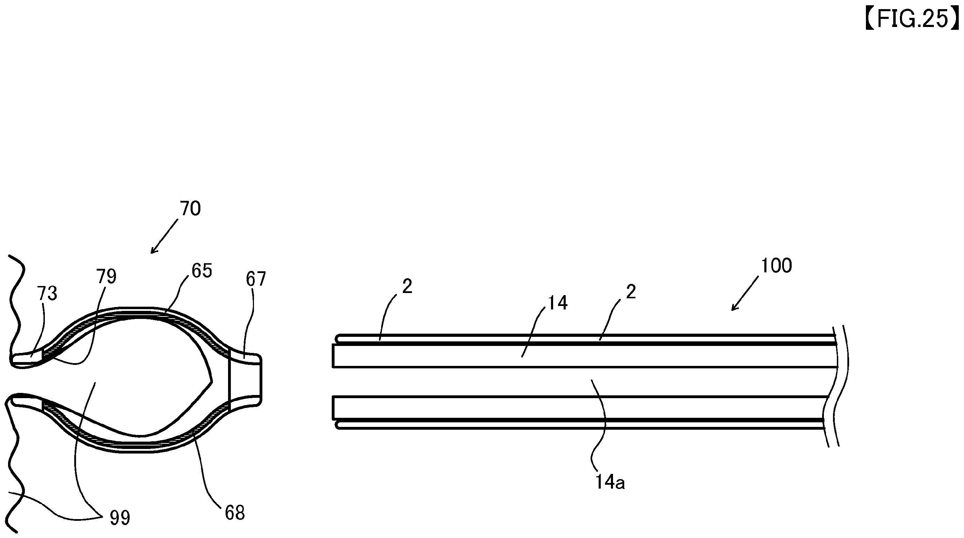

Fig. 21 shows a view at a first stage for explaining a step of placing the indwelling device 70 in the body of a patient. Fig. 22 shows a view at a second stage. Fig. 23 shows a view at a third stage. Fig. 24 shows a view at a fourth stage. Fig. 25 shows a view at a final stage.

### (First Stage)

First, the indwelling device 70 is inserted into the catheter lumen 2a of the catheter body 2 of the medical instrument 100. Then, the pusher member 14 is inserted while in abutting contact with the base end of the indwelling device 70. While the indwelling device 70 is pushed with the pusher member 14, the indwelling device 70 and the pusher member 14 are advanced to the vicinity of the tip of the catheter body 2. In this state, the tip of the catheter body 2 is advanced to the vicinity of the affected part 99 of the patient (see Fig. 21).

### (Second Stage)

After the tip of the catheter body 2 is brought into abutting contact with the affected part 99 of the patient, the indwelling device 70 in the catheter lumen 2a is pushed with the pusher member 14 to bring the tip of the indwelling device 70 into abutting contact with the affected part 99 of the patient (see Fig. 22).

### (Third Stage)

Next, the aspirator 104 is actuated to generate negative pressure in the lumen 14a of the pusher member 14 and in the lumen 70a of the indwelling device 70, thereby aspirating a lesion on the affected part 99 through the tip of the indwelling device 70 (see Fig. 23).

### (Fourth Stage)

After the lesion on the affected part 99 is aspirated into the indwelling device 70, the catheter body 2 is pulled with the pusher member 14 in a grasped state, thereby exposing the indwelling device 70 from the opening portion 2b of the catheter body 2 (see Fig. 24). At this time, as the device tip portion 73 of the indwelling device 70 is made of an elastic member, the affected part 99 is compressed with the device tip portion 73 so that the indwelling device 70 does not come off the affected part 99.

As shown in Fig. 24, the indwelling device 70 is a self-expanding device, so that the indwelling device 70 increases in diameter automatically when the indwelling device 70 is exposed from the opening portion 2b of the catheter body 2.

### (Final Stage)

After the indwelling device 70 is exposed from the opening portion 2b of the catheter body 2, the catheter body 2 and the pusher member 14 are separated from the indwelling device 70. Then, the steps are finished (see Fig. 25).

As described above, in the steps of placing the indwelling device 70 in the body of a patient, the indwelling device increases in diameter automatically when the indwelling device is exposed from the opening portion 2b of the catheter body 2. This also applies to the indwelling device 20, the indwelling device 30, the indwelling device 40, the indwelling device 50, the indwelling device 60, and the indwelling device 80.

While the indwelling devices of the embodiments of the present invention have been described above, the present invention is not limited to the foregoing embodiments but can be implemented in various ways changed within a range not deviating from the substance of the invention.

In the indwelling device 80 of the ninth embodiment, for example, the check valve 89 is provided at the device base end portion 87. A check valve may also be provided at the base end of each of the indwelling device 10, the indwelling device 30, the indwelling device 40, the indwelling device 50, the indwelling device 60, and the indwelling device 70.

Each of the diameter-increased portion of the indwelling device 20, that of the indwelling device 30, and that of the indwelling device 40 may have a two-layered structure with a braid and a resin portion covering the braid.

While the medical instrument 100 has been described in each of the foregoing embodiments as an example of a medical instrument for placing an indwelling device in the body of a patient, such a medical instrument is not limited to the medical instrument 100. Any medical instrument is usable for the indwelling device of the present invention, as long as it can be inserted into the body of a patient and it has the function of aspirating a body tissue of the patient into the indwelling device of the present invention and the function of placing the indwelling device of the present invention in the body of the patient.

### Reference Sings List

1, 10, 20, 30, 40, 50, 60, 70, 80 ... Indwelling device
2 ... Catheter body
3, 13, 23, 33, 43, 53, 63, 73 ... Device tip portion
4 ... Pusher member
5, 15, 45, 55, 65 ... Device body
6 ... Connector
19, 39, 79, 89 ... Check valve
25a, 25b, 35a, 35b, 45a, 45b, 45c, 55a, 55b, 55c, 65a, 65b, 65c ... Diameter-increased portion
47, 57, 67, 87 ... Diameter-reduced portion
68 ... Braid
99 ... Affected part
100 ... Medical instrument
104 ... Aspirator

## Claims

1. An indwelling device configured with a hollow tubular body, wherein
the hollow tubular body has an end portion made of an elastic member.

2. The indwelling device according to claim 1, wherein
the hollow tubular body includes a diameter-increased portion provided closer to a different end than the end portion and having an inner diameter larger than the inner diameter of the end portion.

3. The indwelling device according to claim 2, wherein
the hollow tubular body includes a diameter-reduced portion provided closer to the different end than the diameter-increased portion and having an inner diameter smaller than the inner diameter of the diameter-increased portion.

4. The indwelling device according to claim 2 or 3, wherein
the hollow tubular body has an outer shape formed into a streamlined shape as a whole.

5. The indwelling device according to any one of claims 2 to 4, wherein
the diameter-increased portion includes a braid and a resin portion covering the braid.

6. The indwelling device according to claim 5, wherein
the braid forming the diameter-increased portion is at least partially made of a radiopaque material.

7. The indwelling device according to any one of claims 2 to 6, wherein
the diameter-increased portion is reducible in diameter in a housing in which the indwelling device is accommodated and increases in diameter automatically when the indwelling device is separated from the housing.

8. The indwelling device according to any one of claims 1 to 7, comprising a check valve provided at the end portion of the hollow tubular body.

9. The indwelling device according to any one of claims 1 to 8, comprising a check valve provided at an end portion on the opposite side of the end portion of the hollow tubular body.

10. The indwelling device according to any one of claims 1 to 9, wherein
the elastic member is polyurethane elastomer.
